# EUROPEAN PATENT APPLICATION

(11) **EP 1 738 690 A2**
(43) Date of publication of application: **03.01.2007**
(21) Application number: 06013178.6
(22) Date of filing: 27.06.2006
(51) Int. Cl.: A61B 8/14

(54) **Apparatus and method for forming a 3D ultrasound image**

(30) Priority: 28.06.2005 KR 20050056002
(71) Applicant: MEDISON CO., LTD., Kangwon-do 250-870 (KR)
(72) Inventor: Hyun, Dong Gyu Discusser & Medison Bldg, Seoul 135-280 (KP)
(74) Representative: Lorenz, Werner

(57) **Abstract**

The present invention relates to a method of forming an ultrasound image in an ultrasound diagnostic system, including: a) forming a plurality of 2-dimensional ultrasound images based on ultrasound echo signals reflected from a predetermined portion of a target object; b) setting a region of interest (ROI) on each 2-dimensional image; c) extracting images within the ROIs of the 2-dimensional ultrasound images; and d) sequentially superposing the extracted images to form a 3-dimensional ultrasound image.

## Description

The present invention generally relates to an ultrasound diagnostic system, and more particularly to an apparatus and method for forming a 3-dimensional ultrasound image by using 2-dimenstional ultrasound images acquired from a 1-dimensional or 2-dimensional probe in the ultrasound diagnostic system.

An ultrasound diagnostic system has become an important and popular diagnostic tool due to its wide range of applications. Specifically, due to its non-invasive and non-destructive nature, the ultrasound diagnostic system has been extensively used in the medical profession. Modem high-performance ultrasound diagnostic systems and techniques are commonly used to produce two or three-dimensional diagnostic images of internal features of an object (e.g., organs of a human patient). The ultrasound diagnostic system generally uses a wide bandwidth transducer to transmit and receive ultrasound signals. The ultrasound diagnostic system forms images of the internal tissues of a human body by electrically exciting an acoustic transducer element or an array of acoustic transducer elements to generate ultrasound pulses that travel into the body. The ultrasound pulses produce ultrasound echoes since they are reflected from body tissues, which appear as discontinuities to the propagating ultrasound pulses. The various ultrasound echoes return to the transducer and are converted into electrical signals, which are amplified and processed to produce ultrasound data for an image of the tissues. This ultrasound diagnostic system is of significant importance to the medical field since it provides physicians with real-time high-resolution images of internal features of a human anatomy without the need for invasive observation techniques such as surgery.

The above ultrasound diagnostic system includes a probe for reciprocally converting ultrasound signals and electrical signals to acquire the ultrasound image of the tissue of the target object. Such probe includes: a piezo-electric layer for reciprocally converting the electric signals and ultrasound signals; a matching layer for maximally transmitting the ultrasound signals to a target portion in a human body by reducing an impedance difference between the piezo-electric layer and the human body; a lens for focusing the ultrasound signals to a specific point; and a sound absorbing layer for preventing image distortion by shielding the ultrasound signals so as not to be propagated backward to the piezo-electric layer. Typically, most of the ultrasound probes used for clinical purposes includes a plurality of transducers.

The probe may be classified according to various conditions such as the number of transducers, array of transducers, fields of application, etc. The probe may be classified into a single-type probe or a plural-type probe based on the number of transducers.

Further, the plural-type probe may be classified into a 1-dimensional array probe (arrays the transducers on a single axis) or a 2-dimensional array probe (arrays the transducers on a 2-dimensional grid). The 1-dimensional array probe may be classified into a linear probe or a curvilinear probe depending on the shape of array axis.

Since the ultrasound signals are propagated to the target object in a straight manner, only a 2-dimensional slice image of the target object, which is positioned in front of the transducers, can be acquired. Therefore, an operator cannot often accurately examine the target object by using the 2-dimensional array probe. Also, it is impossible to stereoscopically form an entire shape of a fetus or display a moving image of the fetus. Thus, a 3-dimensional probe is used to form a 3-dimensional image of the interior of the human body, specifically a live 3-dimensional image.

However, since the 3-dimensional probe for forming the live 3-dimensional ultrasound image is configured with a plurality of transducers, such a probe is disadvantageous since it is very complex and expensive.

Accordingly, it is highly desirable to form a 3-dimensional ultrasound image without using the 3-dimensional probe.

The present invention seeks to provide an ultrasound diagnostic system for forming a 3-dimensional ultrasound image by superposing a plurality of 2-dimensional ultrasound images acquired through a 1-dimensional or 2-dimensional probe.

In accordance with one aspect of the present invention, there is provided a method of forming an ultrasound image in an ultrasound diagnostic system, which includes the following steps: a) forming a plurality of 2-dimensional ultrasound images based on ultrasound echo signals reflected from a predetermined portion of a target object; b) setting a region of interest (ROI) on each 2-dimensional image; c) extracting images existing within the ROI from the 2-dimensional ultrasound images; and d) sequentially superposing the extracted images to form a 3-dimensional ultrasound image.

In accordance with another aspect of the present invention, there is provided a method of forming an ultrasound image, which includes the following steps: a) acquiring a plurality of 2-dimensional ultrasound images based on ultrasound echo signals; and b) sequentially superposing the 2-dimensional ultrasound images to form a 3-dimensional ultrasound image.

In accordance with yet another aspect of the present invention, there is provided an apparatus for forming an ultrasound image in an ultrasound diagnostic system, including: a first image forming unit for forming a plurality of 2-dimensional ultrasound images based on ultrasound echo signals reflected from a predetermined portion of a target object; a ROI setting unit for setting a region of interest (ROI) on each 2-dimensional image; an image extracting unit for extracting images within the ROI of the 2-dimensional ultrasound images; and a second image forming unit for sequentially superposing the extracted images to form a 3-dimensional ultrasound image.

The above and other objects and features of the present invention will become apparent from the following description of preferred embodiments given in conjunction with the accompanying drawings, in which:
Fig. 1 is a block diagram showing an ultrasound diagnostic system constructed in accordance with the present invention;
Fig. 2 is a flowchart showing an operation of an image processor constructed in accordance with the present invention;
Figs. 3 and 4 are schematic diagrams showing a 3-dimensional ultrasound image formed in real time in accordance with a preferred embodiment of the present invention; and
Fig. 5 is a schematic diagram showing a 3-dimensional ultrasound image formed in real time in accordance with another preferred embodiment of the present invention.

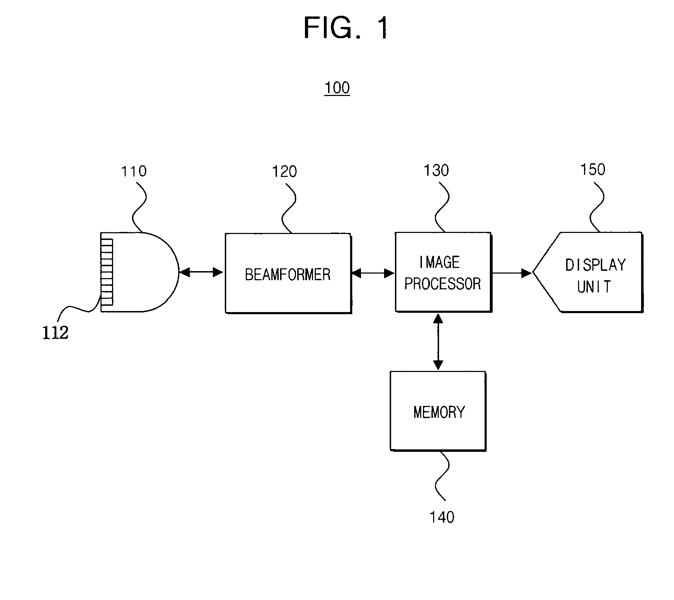

Fig. 1 is a block diagram showing an ultrasound diagnostic system, which is constructed in accordance with the present invention. The ultrasound diagnostic system 100 of the present invention includes a probe 110, a beam former 120, an image processor 130, a memory 140 and a display unit 150.

The probe 110 includes a 1-dimensional or 2-dimensional array transducer 112. The transmit signals, which are appropriately delayed to form a focused ultrasound beam in the beam former 120, are transmitted to the array transducer 112. Then, the focused ultrasound beam is transmitted along a scan line of a target object (not shown). The probe 110 receives ultrasound echo signals reflected from the target object and converts the ultrasound echo signals into electrical signals (hereinafter referred to as receive signals). The receive signals are transmitted into the beam former 120.

The beam former 120 controls the delay of transmit signals to be transmitted to the array transducer 112 in the probe 110 such that the ultrasound signals outputted from the array transducer 112 are focused on a focal point. Further, the beam former 120 focuses the receive signals, which are received at the array transducer 112 included in the probe 110, in consideration of the delays with which the echo signals are arrived at each transducer.

The image processor 130 forms a plurality of 2-dimensional ultrasound images based on the receive signals outputted from the beam former 120 and sequentially superposes a plurality of 2-dimensional ultrasound images to form a 3-dimensional ultrasound image. The 3-dimensional ultrasound image formed in the image processor 130 is displayed on the display unit 150.

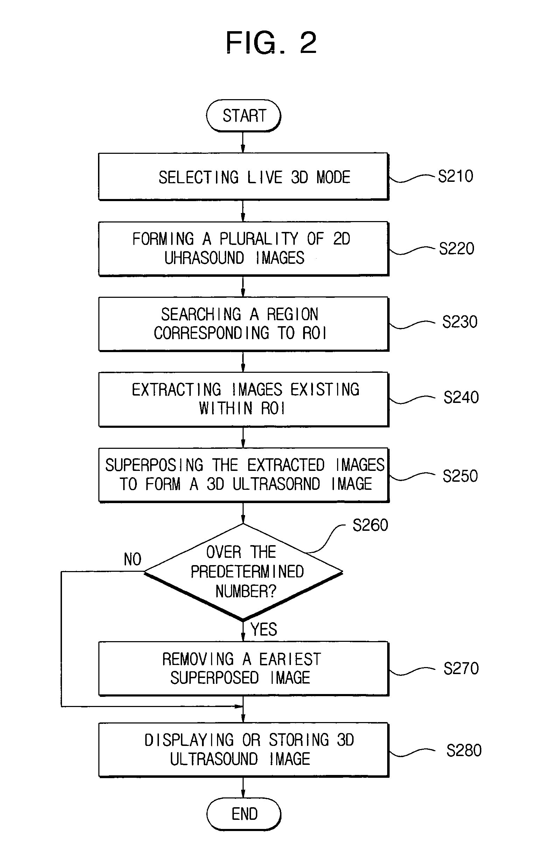

Hereinafter, the operation of the image processor 130 will be described in detail with reference to Figs. 2 to 4. Fig. 2 is a flowchart that shows the operation of the image processor 130, which is constructed in accordance with the present invention. As shown in Fig. 2, if a 3-dimensional mode for forming a live 3-dimensional ultrasound image (hereinafter referred to as "live 3-dimensional mode") is selected by an operator at step S210, then the image processor 130 forms a plurality of 2-dimensional ultrasound images based on the ultrasound echo signals reflected from a predetermined portion of a target object at step S220. Each 2-dimensional ultrasound image may have a serial number, which is assigned in the order of its acquisition time.

The image processor 130 searches a region corresponding to a region of interest (ROI) from the 2-dimensional ultrasound images at step S230. The ROI may be manually set by the operator or may be automatically set according to a preset program. The image processor 130 extracts 2-dimensional images existing within the ROI from the 2-dimensional ultrasound images at step S240. Then, the image processor 130 sequentially superposes the extracted 2-dimensional images, thereby forming a 3-dimensional ultrasound image at step S250.

Subsequently, the image processor 130 checks whether the number of superposed images exceeds a predetermined number at step S260. If it is determined that the number of superposed 2-dimensional images is less than the predetermined number at step S260, then the live 3-dimensional ultrasound image formed in the image processor 130 is displayed on the display unit 150 at step S280.

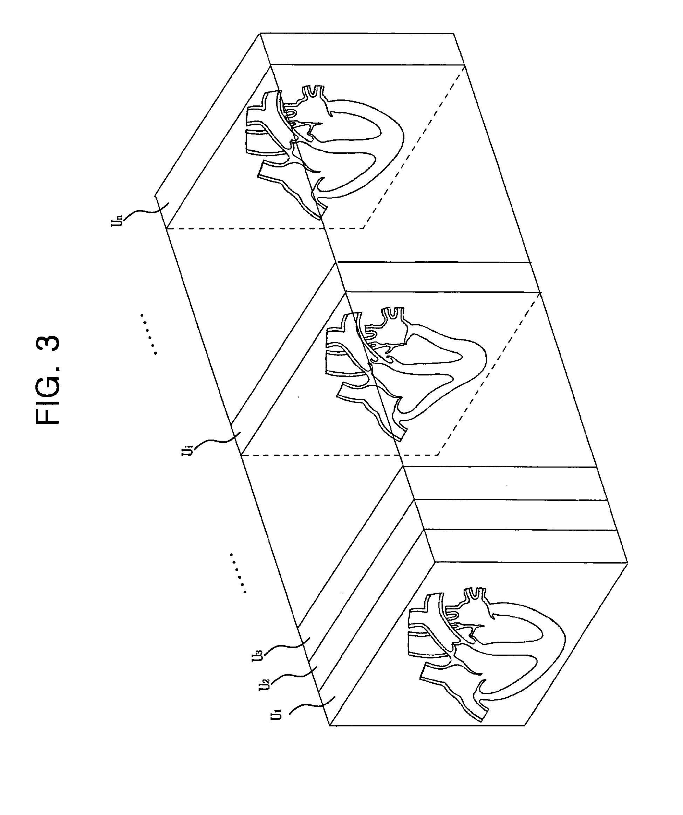

Fig. 3 is a schematic diagram showing the live 3-dimensional ultrasound image formed in real time in accordance with the present invention. In Fig. 3, symbols U₁ to Uₙ represent cardiac slice images. Especially, U₁ and Uₙ represent cardiac slice images of systole, whereas Uᵢ represents a cardiac slice image of diastole.

The image processor 130 forms 2-dimensional ultrasound images, that is, cardiac slice images based on the ultrasound echo signals reflected from the predetermined portion of the target object, and extracts an image U₁ existing within the ROI from the 2-dimensional ultrasound image. Subsequently, the image processor 130 once again forms a 2-dimensional ultrasound image based on the ultrasound echo signals and then extracts an image U₂ within the ROI from the formed 2-dimensional ultrasound image. Thereafter, the extracted image U₂ is superposed to the image U₁, thereby forming the 3-dimensional ultrasound image. In such a case, since the number of superposed images in the 3-dimensional ultrasound image is less than the predetermined number (n), the image processor 130 decides to form the superposed images displayed on the display unit 150.

Subsequently, the image processor 130 extracts an image U₃ within the ROI from the 2-dimensional ultrasound image and then sequentially superposes the extracted image U₃ to the 3-dimensional ultrasound image, which is formed by superposing the images U₁ and U₂. In such a case, since the number of superposed images is also less than n numbers, the superposed images are directly displayed on the display unit 150. The image processor 130 repeatedly extracts and superposes images until the number of superposed images is equal to n. As such, a 3-dimensional ultrasound image, which is formed by superposing images U₁ to Uₙ, can be displayed on the display unit 150, as shown in Fig. 3.

Further, if it is determined that the number of superposed images is greater than the predetermined number n at step S 160, then the image processor 130 removes an image (first superposed among superposed images) from the 3-dimensional ultrasound image at step S270.

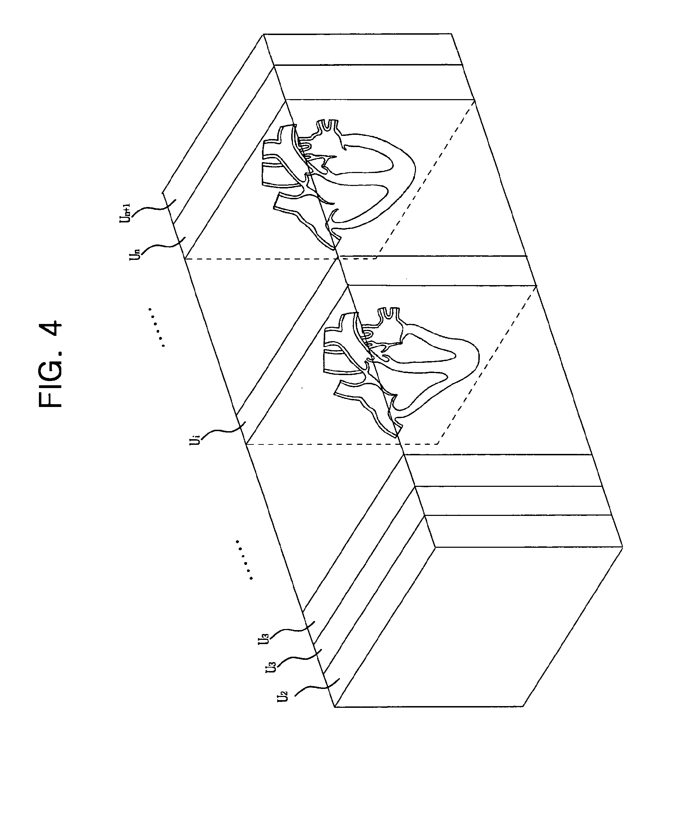

Hereinafter, a process of removing an image from superposed images will be described in detail with reference to Fig. 4. Fig. 4 is a schematic diagram showing a live 3-dimensional ultrasound image, which is formed in accordance with the present invention. In Fig. 4, symbols U₂ to Uₙ₊₁ represent cardiac slice images. Especially, Uᵢ represents a cardiac slice image of diastole (shown in Fig. 3), whereas Uₙ represents a cardiac slice image of systole.

The image processor 130 extracts an image Uₙ₊₁ within the ROI from a 2-dimensional ultrasound image, which is formed based on the ultrasound echo signals.
It then superposes the extracted image Uₙ₊₁ upon the superposed images Uₗ to Uₙ, as illustrated in Fig. 3. In such a case, since the number of superposed images exceeds the predetermined number n, the image U₁ (first extracted among superposed images) is removed form the superposed images. Therefore, the number of superposed images becomes n (U₂ to Uₙ₊₁), as shown in Fig. 4. Subsequently, the image processor 130 allows the superposed images U₂ to Uₙ₊₁ to be displayed as a 3-dimensional ultrasound image on the display unit 150 at step S280.

In the preferred embodiment of the present invention, the 2-dimensional ultrasound images, which are formed based on the ultrasound echo signals reflected from the predetermined portion of the target object, are superposed to form a 3-dimensional ultrasound image in real time. Also, the 2-dimensional ultrasound images may be stored in the memory 140 before forming the 3-dimensional ultrasound image in accordance with another embodiment of the present invention. The image processor 130 reads out the predetermined number of 2-dimensional ultrasound images and superposes them to form a 3-dimensional ultrasound image.

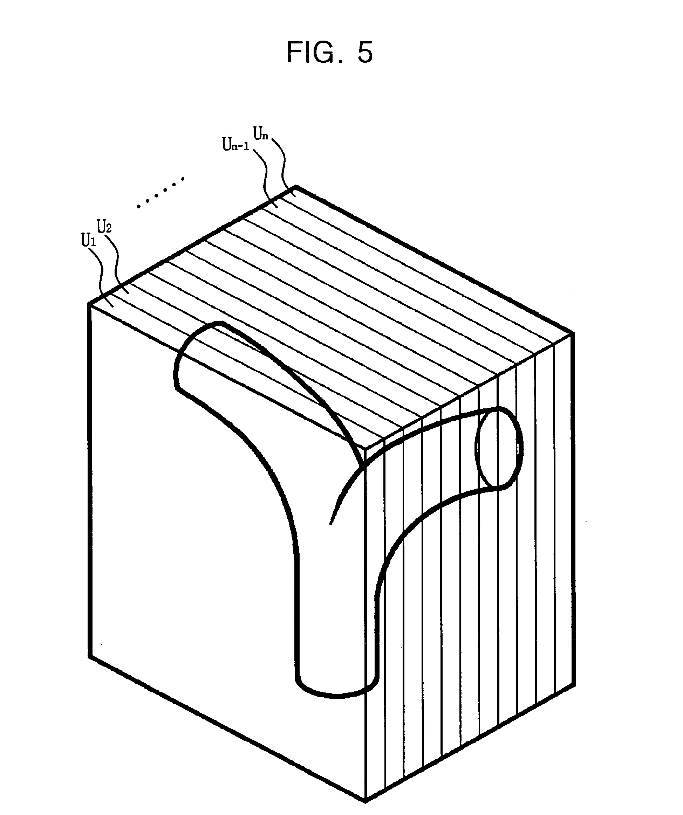

A plurality of 2-dimensional ultrasound images are acquired based on the ultrasound echo signals reflected from the predetermined portion of the target object (e.g., human heart) without moving the probe in accordance with the preferred embodiment of the present invention. Also, the plurality of 2-dimensional ultrasound images can be acquired based on the ultrasound echo signals acquired by swinging the probe within a predetermined range in accordance with another embodiment of the present invention. This is so that a 3-dimensional ultrasound image can be formed, as shown in Fig. 5. The 2-dimensional ultrasound images may be displayed on the display unit 150 together with the 3-dimensional ultrasound image, which is formed in accordance with the present invention.

As discussed above, the 3-dimensional ultrasound image can be formed with the 1-dimensional or 2-dimensional probe in real time in accordance with the present invention, which is highly cost effective compared to using an expensive 3-dimensional probe.

While the present invention has been described and illustrated with respect to a preferred embodiment of the invention, it will be apparent to those skilled in the art that variations and modifications are possible without deviating from the broad principles and teachings of the present invention, which should be limited solely by the scope of the claims appended hereto.

## Claims

1. A method of forming an ultrasound image in an ultrasound diagnostic system, comprising the steps of:
a) forming a plurality of 2-dimensional ultrasound images based on ultrasound echo signals reflected from a predetermined portion of a target object;
b) setting a region of interest (ROI) on each 2-dimensional image;
c) extracting images within the ROIs of the 2-dimensional images; and
d) sequentially superposing the extracted images to form a 3-dimensional ultrasound image.

2. The method of Claim 1, wherein the step a) comprises:
a1) assigning a serial number to each 2-dimensional ultrasound image according to an order of acquisition time of each 2-dimensional ultrasound image.

3. The method of Claim 2, wherein the step a) comprises:
a2) storing the 2-dimensional ultrasound images.

4. The method of Claim 2, wherein the step d) comprises:
d1) determining whether a number of the sequentially superposed images is greater than a predetermined number; and
d2) if it is determined that the number of the superposed images is greater than the predetermined number at the step d1), removing an image having an earliest serial number among the superposed images.

5. A method of forming an ultrasound image, comprising the steps of:
a) acquiring a plurality of 2-dimensional ultrasound images based on ultrasound echo signals; and
b) superposing the 2-dimensional ultrasound images sequentially to form a 3-dimensional ultrasound image.

6. The method of Claim 5, wherein the step a) comprises:
a1) assigning a serial number to each 2-dimensional ultrasound image according to an order of acquisition time of each 2-dimensional ultrasound image.

7. The method of Claim 6, wherein the step a) comprises:
a2) storing the 2-dimensional ultrasound images.

8. The method of Claim 6, wherein the step b) comprises:
d1) determining whether a number of the sequentially superposed images is greater than a predetermined number; and
d2) if it is determined that the number of the superposed images is greater than the predetermined number at the step d1), removing an image having an earliest serial number among the superposed images.

9. An apparatus of forming an ultrasound image in an ultrasound diagnostic system, comprising:
a first image forming unit for forming a plurality of 2-dimensional ultrasound images based on ultrasound echo signals reflected from a predetermined portion of a target object;
a ROI setting unit for setting a region of interest (ROI) on each 2-dimensional image;
an image extracting unit for extracting images within the ROIs of the 2-dimensional ultrasound images; and
a second image forming unit for sequentially superposing the extracted images to form a 3-dimensional ultrasound image.

10. The apparatus of Claim 9, further comprising a storing unit for storing the 2-dimensional ultrasound images.
